# NOUVEAU FASCICULE DE BREVET EUROPEEN

(11) **EP 2 361 605 B2**
(45) Date de publication et mention de la décision concernant l'opposition: **12.03.2025**
(45) Mention de la délivrance du brevet: 24.01.2018
(21) Numéro de dépôt: 10195749.6
(22) Date de dépôt: 17.12.2010
(51) Int. Cl.: A61K 8/31, A61K 8/34, A61K 8/73, A61Q 5/12

(54) **Utilisation pour le traitement cosmétique des fibres keratiniques d'alcane(s) linéaire(s) volatil(s), d'alcool(s) gras solide(s) et d'épaississant(s) polymérique(s) a motifs sucres**
Verwendung von flüchtigem/en linearem/en Alkan/en, festem/en Fettalkohol/en und polymerischem/en Verdickungsmittel/n mit Zucker-Grundeinheiten für die kosmetische Behandlung von Keratinfasern
Use of linear volatile alkane(s), solid fatty alcohol(s) and sugar-based polymeric thickener(s) for the cosmetic treatment of keratin fibres

(30) Priorité: 23.12.2009 FR 0959485
(43) Date de publication de la demande: 31.08.2011
(73) Titulaire: L'OREAL, 75008 Paris (FR)
(72) Inventeur: Desenne, Patricia, 74370 Pringy (FR); Degoul, Marie-Cécile, 75009 Paris (FR)
(74) Mandataire: Casalonga

(56) Documents cités:
- EP-A2- 0 035 899
- DE-A1- 102008 012 457
- US-A- 6 165 455
- US-A1- 2008 269 352

## Description

La présente invention concerne l'utilisation pour le traitement cosmétique des fibres kératiniques, comme les cheveux, d'une composition cosmétique comprenant plus de 1,5 à 10% en poids par rapport au poids total de la composition, d'au moins deux alcanes linéaires volatils distincts différant entre eux d'un nombre de carbones n d'au moins 1, au moins un alcool gras solide et au moins un agent épaississant polymère à motifs sucres.

Dans le domaine du traitement capillaire, l'utilisation de solvants volatils est connue dans des produits capillaires de soins et de brillance. Ils sont généralement utilisés pour différentes raisons. Ils permettent notamment de modifier le rendu sensoriel d'un produit capillaire en lui conférant une texture légère et non collante dans la main. Ils peuvent également lui conférer un caractère glissant qui facilite la répartition du produit sur les cheveux et en particulier sur des cheveux secs.

Ces solvants volatils qui sont généralement des esters gras liquides, des huiles hydrocarbonées de type isododécane ou isohexadécane, et/ou des huiles siliconées, peuvent notamment conduire à des problèmes de toucher gras, de manque de brillance, et de cheveux raidis et durs.

Il subsiste donc un besoin de remplacer ces solvants volatils pour éviter les inconvénients cités ci-dessus.

La demanderesse a maintenant découvert de façon inattendue et surprenante, que l'utilisation d'une composition cosmétique comprenant de 1,5 à 10% en poids par rapport au poids total de la composition, d'au moins deux alcanes linéaires volatils distincts différant entre eux d'un nombre de carbones n d'au moins 1, au moins un alcool gras solide et au moins un agent épaississant polymère à motifs sucres, permettait de surmonter les inconvénients cités ci-dessus et d'améliorer de manière significative les propriétés cosmétiques des cheveux telles que le lissage, la tonicité, le démêlage, la légèreté et la souplesse.

En particulier, lors de l'application de la composition et après rinçage, on obtient des cheveux plus lisses et plus souples.

De plus, cette utilisation conduit notamment à des cheveux humides plus faciles à démêler, plus légers et plus toniques, et à des cheveux séchés plus souples et plus lisses au toucher.

Ainsi, l'invention a pour objet l'utilisation pour le traitement cosmétique des fibres kératiniques, comme les cheveux, d'une composition cosmétique comprenant, dans un milieu cosmétiquement acceptable, de 1,5 à 10% en poids par rapport au poids total de la composition, d'au moins deux alcanes linéaires volatils distincts différant entre eux d'un nombre de carbones n d'au moins 1, au moins un alcool gras solide et au moins un agent épaississant polymère à motifs sucres.

Selon l'invention, une composition cosmétique comprenant, dans un milieu cosmétiquement acceptable :
- de 1,5 à 10% en poids par rapport au poids total de la composition, d'au moins deux alcanes linéaires volatils distincts différant entre eux d'un nombre de carbones n d'au moins 1,
- un ou plusieurs alcools gras solides et
- un ou plusieurs agents épaississants polymères à motifs sucres est utilisée pour le traitement cosmétique des fibres kératiniques, comme les cheveux. Cette utilisation conduit notamment à l'obtention de meilleures propriétés de lissage, de tonicité, de démêlage, de légèreté et/ou de souplesse.

Un autre objet de l'invention est un procédé de traitement cosmétique des matières kératiniques, de préférence des fibres kératiniques comme les cheveux, mettant en oeuvre ladite composition.

Par milieu cosmétiquement acceptable, on entend un milieu compatible avec les matières kératiniques et en particulier les cheveux.

Le milieu cosmétiquement acceptable comprend de préférence de l'eau ou un mélange d'eau et d'un solvant cosmétiquement acceptable choisi parmi les alcools en C₁-C₄, tels que l'éthanol, l'isopropanol, le tertio-butanol ou le n-butanol ; les polyols tels que le glycérol, le propylèneglycol et les polyéthylèneglycols ; et leurs mélanges.

Par « un ou plusieurs alcane(s) linéaire(s) volatil(s) », on entend indifféremment « une ou plusieurs huile alcane(s) linéaire(s) volatile(s) ».

Un alcane linéaire volatil convenant à l'invention est liquide à température ambiante (environ 25 °C) et sous pression atmosphérique (101 325 Pa ou 760 mm Hg).

Par « alcane linéaire volatil » convenant à l'invention, on entend un alcane linéaire susceptible de s'évaporer au contact de la peau en moins d'une heure, à température ambiante (25°C) et sous pression atmosphérique (101 325 Pa), liquide à température ambiante, ayant notamment une vitesse d'évaporation allant de 0,01 à 15 mg/cm²/min, à température ambiante (25°C) et sous pression atmosphérique (101 325 Pa).

De préférence, les alcanes linéaires volatils convenant à l'invention présentent une vitesse d'évaporation allant de 0,01 à 3,5 mg/cm²/min, mieux de 0,01 à 1,5 mg/cm²/min, à température ambiante (25°C) et sous pression atmosphérique (101 325 Pa).

De façon plus préférée, les alcanes linéaires volatils convenant à l'invention présentent une vitesse d'évaporation allant de 0,01 à 0,8 mg/cm²/min, préférentiellement de 0,01 à 0,3 mg/cm²/min, et encore plus préférentiellement de 0,01 à 0,12 mg/cm²/min, à température ambiante (25°C) et sous pression atmosphérique (101 325 Pa).

La vitesse d'évaporation d'un alcane volatil conforme à l'invention (et plus généralement d'un solvant volatil) peut être notamment évaluée au moyen du protocole décrit dans WO 06/013413, et plus particulièrement au moyen du protocole décrit ci-après.

On introduit dans un cristallisoir (diamètre : 7 cm) placé sur une balance se trouvant dans une enceinte d'environ 0,3 m³ régulée en température (25 °C) et en hygrométrie (humidité relative 50 %), 15 g de solvant hydrocarboné volatil.

On laisse le solvant hydrocarboné volatil s'évaporer librement, sans l'agiter, en assurant une ventilation par un ventilateur (PAPST-MOTOREN, référence 8550 N, tournant à 2700 tours/minute) disposé en position verticale au-dessus du cristallisoir contenant le solvant hydrocarboné volatil, les pales étant dirigées vers le cristallisoir, à une distance de 20 cm par rapport au fond du cristallisoir.

On mesure à intervalles de temps réguliers la masse de solvant hydrocarboné volatil restante dans le cristallisoir.

On obtient alors le profil d'évaporation du solvant en traçant la courbe de la quantité de produit évaporé (en mg/cm²) en fonction du temps (en min).

Puis on calcule la vitesse d'évaporation qui correspond à la tangente à l'origine de la courbe obtenue. Les vitesses d'évaporation sont exprimées en mg de solvant volatil évaporé par unité de surface (cm²) et par unité de temps (minute).

Selon un mode de réalisation préféré, les alcanes linéaires volatils convenant à l'invention ont une pression de vapeur (appelée également pression de vapeur saturante) non nulle, à température ambiante, en particulier une pression de vapeur allant de 0,3 Pa à 6000 Pa.

De façon préférée, les alcanes linéaires volatils convenant à l'invention ont une pression de vapeur allant de 0,3 à 2000 Pa, mieux de 0,3 à 1000 Pa, à température ambiante (25°C).

De façon plus préférée, les alcanes linéaires volatils convenant à l'invention ont une pression de vapeur allant de 0,4 à 600 Pa, préférentiellement de 1 à 200 Pa, et encore plus préférentiellement de 3 à 60 Pa, à température ambiante (25°C).

Selon un mode de réalisation, un alcane linéaire volatil convenant à l'invention peut présenter un point éclair compris dans l'intervalle variant de 30 à 120 °C, et plus particulièrement de 40 à 100 °C. Le point éclair est en particulier mesuré selon la Norme iso 3679.

Selon un mode de réalisation, les alcanes linéaires volatils convenant dans l'invention peuvent être les alcanes linéaires comportant de 7 à 15 atomes de carbone, de préférence de 8 à 14 atomes de carbone et mieux de 9 à 14 atomes de carbone.

De façon plus préférée, les alcanes linéaires volatils convenant dans l'invention peuvent être les alcanes linéaires comportant de 10 à 14 atomes de carbone, et encore plus préférentiellement de 11 à 14 atomes de carbone.

Un alcane linéaire volatil convenant à l'invention peut être avantageusement d'origine végétale.

De préférence, l'alcane linéaire volatil ou le mélange d'alcanes linéaires volatils présent dans la composition selon l'invention comprend au moins un isotope ¹⁴C du carbone (carbone 14). En particulier, l'isotope ¹⁴C peut être présent en un rapport en nombre d'isotopes ¹⁴C/¹²C (ou ratio ¹⁴C/¹²C) supérieur ou égal à 1.10⁻¹⁶, de préférence supérieur ou égal à 1.10⁻¹⁵, de préférence encore supérieur ou égal 7,5.10⁻¹⁴, et mieux supérieur ou égal 1,5.10⁻¹³. De préférence, le ratio ¹⁴C / ¹²C va de 6.10⁻¹³ à 1,2.10⁻¹²

La quantité d'isotopes ¹⁴C dans l'alcane linéaire volatil ou le mélange d'alcanes linéaires volatils peut être déterminée par des méthodes connues de l'homme du métier telles que la méthode de comptage de Libby, la spectrométrie à scintillation liquide ou encore la spectrométrie de masse à accélération (Accelerator Mass Spectrometry).

Un tel alcane peut être obtenu, directement ou en plusieurs étapes, à partir d'une matière première végétale comme une huile, un beurre, une cire, etc.

A titre d'exemple d'alcanes convenant à l'invention, on peut mentionner les alcanes décrits dans les demandes de brevets WO 2007/068371 et WO2008/155059. Ces alcanes sont obtenus à partir d'alcools gras, eux-mêmes obtenus à partir d'huile de coprah ou de palme.

A titre d'exemples d'alcanes linéaires volatils convenant à l'invention, on peut citer le n- heptane (C7), le n-octane (C8), le n-nonane (C9), le n-décane (C10), le n-undécane (C11), le n-dodécane (C12), le n-tridécane (C13), le n-tétradécane (C14), le n-pentadécane (C15), et leurs mélanges. Selon un mode de réalisation particulier, l'alcane linéaire volatil est choisi parmi le n-nonane, le n-undécane, le n-dodécane, le n-tridécane, le n-tétradécane, et leurs mélanges.

Selon un mode de réalisation préféré, on peut citer les mélanges de n-undécane (C11) et de n-tridécane (C13) obtenus aux exemples 1 et 2 de la demande WO2008/155059.

On peut également citer le n-dodécane (C12) et le n-tétradécane (C14) vendus respectivement sous les références PARAFOL 12-97 et PARAFOL 14-97 par la société Sasol, ainsi que leurs mélanges. Selon l'invention on utilise un mélange d'au moins deux alcanes linéaires volatils distincts, différant entre eux d'un nombre de carbone n d'au moins 1, en particulier différant entre eux d'un nombre de carbone de 1 ou de 2.

Selon un mode de réalisation, on utilise un mélange d'au moins deux alcanes linéaires volatils distincts comportant de 10 à 14 atomes de carbone et différant entre eux d'un nombre de carbone d'au moins 1. A titre d'exemples, on peut citer notamment les mélanges d'alcanes linéaires volatils C10/C11, C11/C12, ou C12/C13.

Selon un autre mode de réalisation, on utilise un mélange d'au moins deux alcanes linéaires volatils distincts comportant de 10 à 14 atomes de carbone et différant entre eux d'un nombre de carbone d'au moins 2. A titre d'exemples, on peut citer notamment les mélanges d'alcanes linéaires volatils C10/C12, ou C12/C14, pour un nombre de carbone n pair et le mélange C11/C13 pour un nombre de carbone n impair.

Selon un mode de réalisation préféré, on utilise un mélange d'au moins deux alcanes linéaires volatils comportant de 10 à 14 atomes de carbone distincts et différant entre eux d'un nombre de carbone d'au moins 2, et en particulier un mélange d'alcanes linéaires volatils C11/C13 ou un mélange d'alcanes linéaires volatils C12/C14.

D'autres mélanges associant plus de 2 alcanes linéaires volatils selon l'invention, tels que, par exemple, un mélange d'au moins 3 alcanes linéaires volatils comportant de 7 à 15 atomes de carbone distincts et différant entre eux d'un nombre de carbone d'au moins 1, peuvent être utilisés dans l'invention.

Dans les mélanges de deux alcanes linéaires volatils, lesdits deux alcanes linéaires volatils représentent de préférence plus de 95% et mieux plus de 99% en poids du mélange.

Selon un mode particulier de l'invention, dans un mélange d'alcanes linéaires volatils, l'alcane linéaire volatil ayant le nombre de carbone le plus petit est majoritaire dans le mélange.

Selon un autre mode de l'invention, on utilise un mélange d'alcanes linéaires volatils dans lequel l'alcane linéaire volatil ayant le nombre de carbone le plus grand est majoritaire dans le mélange.

A titre d'exemples de mélanges convenant à l'invention, on peut citer notamment les mélanges suivants :
- de 50 à 90% en poids, de préférence de 55 à 80% en poids, préférentiellement encore de 60 à 75% en poids d'alcane linéaire volatil en Cₙ avec n allant de 7 à 15
- de 10 à 50% en poids, de préférence de 20 à 45% en poids, de préférence de 24 à 40% en poids, d'alcane linéaire volatil en Cₙ₊ₓ avec x supérieur ou égal à 1, de préférence x=1 ou x=2, avec n+x compris entre 8 et 14,
par rapport au poids total des alcanes dans ledit mélange.

En particulier, ledit mélange d'alcanes linéaires volatils peut contenir en outre :
- moins de 2% en poids, de préférence moins de 1% en poids d'hydrocarbures ramifiés,
- et/ou moins de 2% en poids, de préférence moins de 1% en poids d'hydrocarbures aromatiques,
- et/ou moins de 2% en poids, de préférence moins de 1% en poids et préférentiellement moins de 0,1% en poids d'hydrocarbures insaturés,
lesdits pourcentages étant exprimés par rapport au poids total du mélange.

Plus particulièrement, les alcanes linéaires volatils convenant dans l'invention peuvent être mis en oeuvre sous la forme d'un mélange n-undécane/n-tridécane.

En particulier, on utilisera un mélange d'alcanes linéaires volatils comprenant :
- de 55 à 80% en poids, de préférence de 60 à 75% en poids d'alcane linéaire volatil en C11 (n-undécane) et
- de 20 à 45% en poids, de préférence de 24 à 40% en poids d'alcane linéaire volatil en C13 (n-tridécane),
par rapport au poids total des alcanes dans ledit mélange.

Selon un mode de réalisation particulier, le mélange d'alcanes est un mélange n-undécane/n-tridécane. En particulier, un tel mélange peut être obtenu selon l'exemple 1 ou l'exemple 2 de la demande WO 2008/155059.

Selon un autre mode de réalisation particulier, on utilise le n-dodécane vendu sous la référence PARAFOL 12-97 par SASOL.

Selon un autre mode de réalisation particulier, on utilise le n-tétradécane vendu sous la référence PARAFOL 14-97 par SASOL.

Selon encore un autre mode de réalisation, on utilise un mélange de n-dodécane et de n-tétradécane.

Les alcanes linéaires volatils sont utilisés en une quantité préférentielle de 2 à 10 % en poids par rapport au poids total de la composition.

Par « alcool gras solide », on entend au sens de la présente invention, un composé de formule R-OH, R désignant une chaîne alkyle ou alcényle en C₁₀-C₃₀, linéaire ou ramifié, solide à température ambiante (25°C) et à pression atmosphérique (101 325 Pa).

De préférence R désigne une chaîne alkyle linéaire en C₁₀-C₃₀.

Le ou les alcool(s) gras solide (s) utilisé(s) dans l'invention sont de préférence des alcools gras linéaires en C₁₂₋₃₀, mieux en C₁₂₋₂₆.

Comme exemples d'alcools gras solides pouvant être utilisés dans la présente invention, on peut notamment citer les alcools myristylique, cétylique, stéarylique, cétéarylique, arachidylique, béhénylique, lignocérylique, cérylique et montanylique et leurs mélanges.

De préférence, l'alcool gras solide utilisé dans l'invention est choisi parmi les alcools myristylique, cétylique, stéarylique, cétéarylique, béhénylique, et leurs mélanges.

Le ou les alcools gras solides peuvent être utilisés en une quantité allant de 0,5 à 20 % en poids, de préférence de 1 à 15% en poids, et plus particulièrement de 2 à 10% en poids, par rapport au poids total de la composition.

Au sens de la présente invention, on entend par agent épaississant polymère, un polymère qui introduit à 1% en poids dans une solution aqueuse ou hydroalcoolique à 30 % d'éthanol, et à pH = 7, permet d'atteindre une viscosité d'au moins 0,1 Pa.s (100 cps), de préférence au moins 0,5 Pa.s (500 cps), à 25°C et à un taux de cisaillement de 1 s⁻¹. Cette viscosité peut être mesurée à l'aide d'un viscosimètre cône/plan (Rhéomètre Haake R600 ou analogue).

Par motif sucre, on entend au sens de la présente invention un motif issu d'un hydrate de carbone de formule Cₙ(H₂O)ₙ₋₁ ou (CH₂O)ₙ pouvant être éventuellement modifié par substitution, et/ou par oxydation et/ou par déshydratation.

Les motifs sucre pouvant entrer dans la formule chimique des agents épaississants polymères de l'invention sont de préférence issus des sucres suivants :
- glucose
- galactose
- arabinose
- rhamnose
- mannose
- xylose
- fucose
- anhydro galactose
- acide galacturonique
- acide glucuronique
- acide mannuronique
- galactose sulfate
- anhydrogalactose sulfate

Les agents épaississants polymères à motifs sucre de l'invention peuvent être des polysaccharides naturels ou synthétiques.

Ils peuvent être non-ioniques, anioniques, amphotères ou cationiques.

On peut notamment citer à titre d'agents épaississants polymères à motifs sucres utilisés dans l'invention :
- les gommes natives telles que :
   a) les exsudats d'arbres ou d'arbustes dont :
      - la gomme arabique (polymère ramifié de galactose, d'arabinose, de rhamnose et d'acide glucuronique)
      - la gomme ghatti (polymère issu d'arabinose, de galactose, de mannose, de xylose et d'acide glucuronique)
      - la gomme karaya (polymère issu d'acide galacturonique, de galactose, de rhamnose et d'acide glucuronique)
      - la gomme tragacanthe (ou adragante) (polymère d'acide galacturonique, de galactose, de fucose, de xylose et d'arabinose)
   b) les gommes issues d'algues dont :
      - l'agar (polymère issu de galactose et d'anhydrogalactose)
      - les alginates (polymères d'acide mannuronique et d'acide glucuronique)
      - les carraghénanes et les furcelleranes (polymères de galactose sulfate et d'anhydrogalactose sulfate)
   c) les gommes issus de semences ou tubercules dont :
      - la gomme de guar (polymère de mannose et de galactose)
      - la gomme de caroube (polymère de mannose et de galactose)
      - la gomme de fenugrec (polymère de mannose et de galactose)
      - la gomme de tamarin (polymère de galactose, de xylose et de glucose)
      - la gomme de konjac (polymère de glucose et mannose)
      - l'amidon (polymère du glucose)
   d) les gommes microbiennes dont :
      - la gomme de xanthane (polymère de glucose, de mannose acétate, de mannose/acide pyruvique et d'acide glucuronique)
      - la gomme de gellane (polymère de glucose partiellement acylé, de rhamnose et d'acide glucuronique)
      - la gomme de scléroglucane (polymère du glucose)
   e) les extraits de plantes dont :
      - la cellulose (polymère du glucose, motif de répétition cellobiose)
      - l'amidon (polymère du glucose, motifs de répétition amylose et amylopectine).

Ces gommes natives peuvent être modifiées par voie physique ou chimique. A titre de traitement physique, on peut citer notamment la température.

A titre de traitements chimiques, on peut citer les réactions d'estérification, d'étherification, d'amidification, d'oxydation. Ces traitements permettent de conduire à des polymères qui peuvent être non ioniques, anioniques, cationiques ou amphotères, et de préférence non-ioniques ou cationiques.

De préférence, ces traitements chimiques ou physiques sont appliqués sur les gommes de guar, les gommes de caroube, les amidons et les celluloses.

Dans un mode de réalisation de l'invention, l'agent épaississant polymère à motifs sucres est choisi parmi les gommes de guar non-ioniques modifiées par des groupements hydroxyalkyle en C₁-C₆, les gommes de guar modifiées par des groupements cationiques, la gomme de caroube modifiée par des groupements hydroxypropyltrimmonium, les amidons prégélatinisés et/ou oxydés et/ou réticulés et/ou estérifiés, les amidons amphotères, les éthers de celluloses, les esters de celluloses et les esters éthers de celluloses, et leurs mélanges.

Les gommes de guar non-ioniques utilisables selon l'invention peuvent être modifiées par des groupements hydroxylakyle en C₁-C₆.

Parmi les groupements hydroxyalkyle, on peut mentionner à titre d'exemple, les groupements hydroxyméthyle, hydroxyéthyle, hydroxypropyle et hydroxybutyle.

Ces gommes de guar sont bien connues de l'état de la technique et peuvent, par exemple, être préparées en faisant réagir des oxydes d'alcènes correspondants tel que par exemple des oxydes de propylène avec la gomme de guar de façon à obtenir une gomme de guar modifiée par des groupements hydroxypropyle.

Le taux d'hydroxyalkylation varie de préférence de 0,4 à 1,2 et correspond au nombre de molécules d'oxyde d'alkylène consommé par le nombre de fonctions hydroxyle libres présentes sur la gomme de guar.

De telles gommes de guar non-ioniques éventuellement modifiées par des groupements hydroxyalkyle sont par exemple vendues sous les dénominations commerciales JAGUAR HP8, JAGUAR HP60, JAGUAR HP105 et JAGUAR HP120 par la société RHODIA CHIMIE.

Les gommes de guar modifiées par des groupements cationiques plus particulièrement utilisables selon l'invention sont des gommes de guar comportant des groupements cationiques trialkylammonium. De préférence, 2 à 30 % en nombre des fonctions hydroxyle de ces gommes de guar porte des groupements cationiques trialkyammonium. Encore plus préférentiellement, 5 à 20 % du nombre des fonctions hydroxyle de ces gommes de guar portent des groupements cationiques trialkyammonium.

Parmi ces groupements trialkylammonium, on peut tout particulièrement citer les groupements triméthylammonium et triéthylammonium.

Encore plus préférentiellement, ces groupements représentent de 5 à 20 % en poids par rapport au poids total de la gomme de guar modifiée.

Selon l'invention, on peut utiliser des gommes de guar modifiées par du chlorure de 2,3-époxypropyl triméthylammonium.

Ces gommes de guar modifiées par des groupements cationiques sont des produits déjà connus en eux-mêmes et sont par exemple décrits dans les brevets US 3 589 578 et US 4 0131 307. De tels produits sont par ailleurs vendus notamment sous les dénominations commerciales de JAGUAR C13 S, JAGUAR C 15, JAGUAR C 17 par la société RHODIA CHIMIE.

A titre de gomme de caroube modifiée, on peut utiliser la gomme de caroube cationique contenant des groupements hydroxypropyltrimmonium telle que le Catinal CLB 200 proposé par la société TOHO.

Les molécules d'amidons utilisées dans la présente invention peuvent avoir comme origine botanique les céréales ou encore les tubercules. Ainsi, les amidons sont par exemple choisis parmi les amidons de maïs, de riz, de manioc, d'orge, de pomme de terre, de blé, de sorgho, de pois.

Les amidons peuvent être modifiés par voie chimique ou physique, notamment par une ou plusieurs des réactions suivantes : prégélatinisation, oxydation, réticulation, estérification, éthérification, amidification, traitements thermiques.

De manière plus particulière, ces réactions peuvent être réalisées de la façon suivante :
- prégélatinisation en faisant éclater les granules d'amidon (par exemple séchage et cuisson dans un tambour sécheur) ;
- oxydation par des oxydants forts conduisant à l'introduction de groupes carboxyle dans la molécule d'amidon et à la dépolymérisation de la molécule d'amidon (par exemple en traitant une solution aqueuse d'amidon par l'hypochlorite de sodium) ;
- réticulation par des agents fonctionnels capables de réagir avec les groupes hydroxyle des molécules d'amidon qui vont ainsi être liées entre elles (par exemple avec des groupes glycéryl et/ou phosphate) ;
- estérification en milieu alcalin pour le greffage de groupes fonctionnels, notamment acyl en C₁-C₆ (acétyl), hydroxyalkylés en C₁-C₆ tels que hydroxyéthyl et hydroxypropyl, carboxyméthyl, octénylsuccinique.

On peut notamment obtenir par réticulation avec des composés phophorés, des phosphates de monoamidon (du type Am-O-PO-(OX)₂), des phosphates de diamidon (du type Am-O-PO-(OX)-O-Am) ou même de triamidon (du type Am-O-PO-(O-Am)₂) ou leurs mélanges, Am signifiant amidon.

X désigne notamment les métaux alcalins (par exemple sodium ou potassium), les métaux alcalinoterreux (par exemple calcium, magnésium), les sels d'ammoniaque, les sels d'amines comme ceux de la monoéthanolamine, la diéthanolamine, la triéthanolamine, le 3-amino-1,2-propanediol, les sels ammoniums issus des aminoacides basiques tels que la lysine, l'arginine, la sarcosine, l'ornithine, la citrulline.

Les composés phosphorés peuvent être par exemple du tripolyphosphate de sodium, de l'orthophosphate de sodium, de l'oxychlorure de phosphore ou du trimétaphosphate de sodium.

On utilisera préférentiellement des phosphates de diamidon ou des composés riches en phosphate de diamidon comme le produit proposé sous les références PREJEL VA-70-T AGGL (phosphate de diamidon de manioc hydroxypropylé gélatinisé) ou PREJEL TK1 (phosphate de diamidon de manioc gélatinisé) ou PREJEL 200 (phosphate de diamidon de manioc acétylé gélatinisé) par la Société AVEBE ou STRUCTURE ZEA de NATIONAL STARCH (phosphate de diamidon de maïs gélatinisé).

Un amidon préféré est un amidon ayant subi au moins une modification voie chimique telle qu'au moins une estérification.

Selon l'invention, on peut aussi utiliser des amidons amphotères, ces amidons amphotères comprennent un ou plusieurs groupements anioniques et un ou plusieurs groupements cationiques. Les groupements anioniques et cationiques peuvent être liés au même site réactif de la molécule d'amidon ou à des sites réactifs différents; de préférence ils sont liés au même site réactif. Les groupements anioniques peuvent être de type carboxylique, phosphate ou sulfate et de préférence carboxylique. Les groupements cationiques peuvent être de type amine primaire, secondaire, tertiaire ou quaternaire.

Les amidons amphotères sont notamment choisis parmi les composés de formules suivantes : 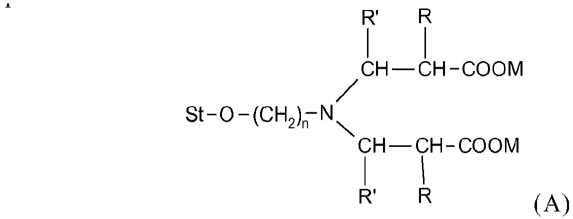 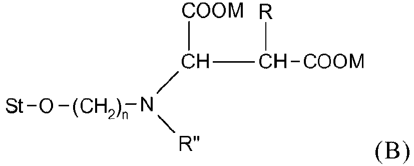 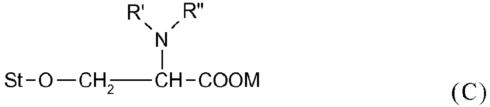 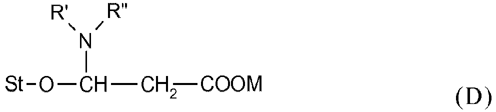 formules dans lesquelles :
St-O représente une molécule d'amidon,
R, identique ou différent, représente un atome d'hydrogène ou un groupe méthyle,
R', identique ou différent, représente un atome d'hydrogène, un groupe méthyle ou un groupement -COOH,
n est un entier égal à 2 ou 3,
M, identique ou différent, désigne un atome d'hydrogène, un métal alcalin ou alcalinoterreux tels que Na, K, Li, NH₄, un ammonium quaternaire ou une amine organique,
R" représente un atome d'hydrogène ou un radical alkyle ayant de 1 à 18 atomes de carbone.

Ces composés sont notamment décrits dans les brevets US 5,455,340 et US 4,017,460.

Les molécules d'amidons peuvent être issues de toutes les sources végétales d'amidon telles que notamment le maïs, la pomme de terre, l'avoine, le riz, le tapioca, le sorgho, l'orge ou le blé. On peut également utiliser les hydrolysats des amidons cités ci-dessus. L'amidon est de préférence issu de la pomme de terre.

On utilise particulièrement les amidons de formules (B) ou (C). On utilise plus particulièrement les amidons modifiés par de l'acide 2-chloroéthylaminodipropionique, c'est-à-dire les amidons de formule (B) ou (C) dans lesquelles R, R', R" et M représentent un atome d'hydrogène et n est égal à 2. L'amidon amphotère préféré est un chloroéthylamidodipropionate d'amidon.

On peut aussi utiliser comme agent épaississant polymère à motifs sucres, des dérivés de celluloses comme celluloses modifiées. Ils peuvent être anioniques, cationiques, amphotères ou non-ioniques.

Les dérivés de cellulose peuvent être des éthers de celluloses, des esters de celluloses ou des esters éthers de celluloses.

Parmi les esters de celluloses, on trouve les esters inorganiques de cellulose (nitrates, sulfates ou phosphates de cellulose...), les esters organiques de cellulose (monoacétates, triacétates, amidopropionates, acétatebutyrates, acétatepropionates ou acétatetrimellitates de cellulose....) et les esters mixtes organique/inorganique de cellulose tels que les acétatebutyratesulfates et les acétatepropionatesulfates de cellulose. Parmi les esters éthers de cellulose, on peut citer les phtalates d'hydroxypropylméthylcellulose et les sulftates d'éthylcellulose.

Parmi les éthers de cellulose non ioniques, on peut citer les alkylcelluloses telles que les méthylcelluloses et les éthylcelluloses (par exemple Ethocel standard 100 Premium de DOW CHEMICAL) ; les hydroxyalkylcelluloses telles que les hydroxyméthylcelluloses, les hydroxyéthylcelluloses (par exemple Natrosol 250 HHR proposé par AQUALON) et les hydroxypropylcelluloses (par exemple Klucel EF d'AQUALON); les celluloses mixtes hydroxyalkyl-alkylcelluloses telles que les hydroxypropyl-méthylcelluloses (par exemple Methocel E4M de DOW CHEMICAL), les hydroxyéthyl-méthylcelluloses, les hydroxyéthyl-éthylcelluloses (par exemple Bermocoll E 481 FQ d'AKZO NOBEL) et les hydroxybutyl-méthylcelluloses.

Parmi les éthers de cellulose anioniques, on peut citer les carboxyalkylcelluloses et leurs sels. A titre d'exemples, on peut citer les carboxyméthylcelluloses, les carboxyméthylméthylcelluloses (par exemple Blanose 7M de la société AQUALON) et les carboxyméthylhydroxyéthylcelluloses et leurs sels de sodium.

Parmi les éthers de cellulose cationiques, on peut citer les hydroxyéthylcelluloses quaternisées réticulées ou non. L'agent quaternisant peut être notamment le chlorure de diallyldiméthylammonium (par exemple Celquat L200 de NATIONAL STARCH). Comme autre éther de cellulose cationique, on peut citer l'hydroxyéthylcellulose hydroxypropyltriméthylammonium (par exemple Ucare polymer JR 400 d'AMERCHOL).

Comme autres agents épaississants polymères utilisables dans l'invention on peut citer les polymères associatifs choisis notamment parmi les celluloses ou les gommes de guar modifiées chimiquement. Des exemples de celluloses modifiées chimiquement sont :
- les celluloses ou leurs dérivés, modifiés par des groupements comportant au moins une chaîne grasse tels que des groupes alkyles, arylalkyles, alkylaryles ou leurs mélanges où les groupes alkyles sont en C₈-C₂₂ ;
- les alkylhydroxyéthylcelluloses non-ioniques telles que les produits NATROSOL PLUS GRADE 330 CS et POLYSURF 67 (alkyle en C16) vendus par la société AQUALON ;
- les alkylhydroxyéthylcelluloses quaternisées (cationiques) telles que les produits QUATRISOFT LM 200, QUATRISOFT LM-X 529-18-A, QUATRISOFT LM-X 529-18-B (alkyle en C12) et QUATRISOFT LM-X 529-8 (alkyle en C18) vendus par la société AMERCHOL, les produits CRODACEL QM , CRODACEL QL (alkyle en C12) et CRODACEL QS (alkyle en C18) vendus par la société CRODA et le produit SOFTCAT SL 100 vendu par la société AMERCHOL.
- les nonoxynylhydroxyéthylcelluloses non-ioniques tels que le produit AMERCELL HM-1500 vendu par la société AMERCHOL ;
- les alkylcelluloses non-ioniques telles que le produit BERMOCOLL EHM 100 vendu par la société BEROL NOBEL.

A titre de dérivés de guar associatifs, on peut utiliser les hydroxypropylguars modifiés par une chaîne grasse tel que le produit ESAFLOR HM 22 (modifié par une chaîne alkyle en C22) vendu par la société LAMBERTI ; le produit MIRACARE XC 95-3 (modifié par une chaîne alkyle en C14) et le produit RE 205-146 (modifié par une chaîne alkyle en C20) vendus par RHODIA CHIMIE.

Le ou les agents épaississants polymères à motifs sucres utilisés dans l'invention sont choisis de préférence parmi les gommes de guar, les amidons et les celluloses, modifiés ou non.

De préférence, les agents épaississants polymères à motifs sucres de l'invention sont non-ioniques ou cationiques.

Le ou les agent(s) épaississant(s) polymère(s) à motifs sucres est ou sont de préférence utilisé(s) en une quantité allant de 0,01 à 10% en poids, encore plus préférentiellement de 0,1 à 5% en poids, et mieux encore de 0,2 à 3% en poids par rapport au poids de la composition.

La composition utilisée dans la présente invention peut contenir en outre au moins un agent tensioactif cationique.

A titre d'exemples d'agent tensioactif cationique, on peut notamment citer les sels d'amines grasses primaires, secondaires ou tertiaires, éventuellement polyoxyalkylénées ; les sels d'ammonium quaternaire ; les dérivés d'imidazoline ; ou les oxydes d'amines à caractère cationique.

Comme exemples de sels d'amines grasses primaires, secondaires ou tertiaires, éventuellement polyoxyalkylénées, on peut notamment citer la distéarylamine.

A titre de sels d'ammonium quaternaires, on peut notamment citer, par exemple :
- ceux qui présentent la formule générale (I) suivante : 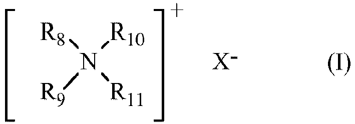 dans laquelle les radicaux R₈ à R₁₁, qui peuvent être identiques ou différents, représentent un radical aliphatique, linéaire ou ramifié, comportant de 1 à 30 atomes de carbone, ou un radical aromatique tel que aryle ou alkylaryle, au moins un des radicaux R₈ à R₁₁ ayant de 8 à 30 atomes de carbone, de préférence de 12 à 24 atomes de carbone. Les radicaux aliphatiques peuvent comporter des hétéroatomes tels que notamment l'oxygène, l'azote, le soufre et les halogènes. Les radicaux aliphatiques sont par exemple choisis parmi les radicaux alkyle, alcoxy, polyoxyalkylène (C₂-C₆), alkylamide, alkyl(C₁₂-C₂₂)amidoalkyle(C₂-C₆), alkyl(C₁₂-C₂₂)acétate, hydroxyalkyle, comportant environ de 1 à 30 atomes de carbone ; X est un anion choisi dans le groupe des halogénures, phosphates, acétates, lactates, alkyl(C₂-C₆)sulfates, alkyl-ou alkylaryl-sulfonates.

Parmi les sels d'ammonium quaternaire de formule (I), on préfère d'une part, les chlorures de tétraalkylammonium comme, par exemple, les chlorures de dialkyldiméthylammonium ou d'alkyltrimé-thylammonium dans lesquels le radical alkyle comporte environ de 12 à 22 atomes de carbone, en particulier les chlorures de béhényltriméthyl-ammonium, de distéaryldiméthylammonium, de cétyltriméthylammo-nium, de benzyldiméthylstéarylammonium ou encore, d'autre part, le chlorure de palmitylamidopropyltriméthylammonium ou le chlorure de stéaramidopropyldiméthyl-(myristyl acétate)-ammonium commercialisé sous la dénomination CERAPHYL^{®} 70 par la société VAN DYK.
- les sels d'ammonium quaternaire de l'imidazoline, comme par exemple ceux de formule (II) suivante : 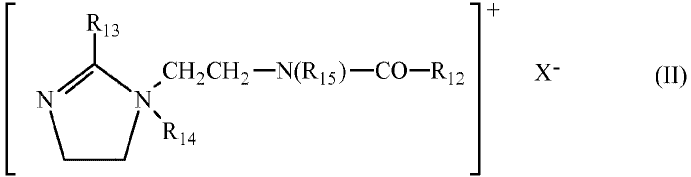 dans laquelle R₁₂ représente un radical alcényle ou alkyle comportant de 8 à 30 atomes de carbone, par exemple dérivés des acides gras du suif, R₁₃ représente un atome d'hydrogène, un radical alkyle en C₁-C₄ ou un radical alcényle ou alkyle comportant de 8 à 30 atomes de carbone, R₁₄ représente un radical alkyle en C₁-C₄, R₁₅ représente un atome d'hydrogène, un radical alkyle en C₁-C₄, X⁻ est un anion choisi dans le groupe des halogénures, phosphates, acétates, lactates, alkylsulfates, alkyl- ou alkylaryl-sulfonates. De préférence, R₁₂ et R₁₃ désignent un mélange de radicaux alcényle ou alkyle comportant de 12 à 21 atomes de carbone, par exemple dérivés des acides gras du suif, R₁₄ désigne un radical méthyle, R₁₅ désigne un atome d'hydrogène. Un tel produit est par exemple commercialisé sous la dénomination REWOQUAT^{®} W 75 par la société REWO ;
- les sels de diammonium quaternaire de formule (III) : 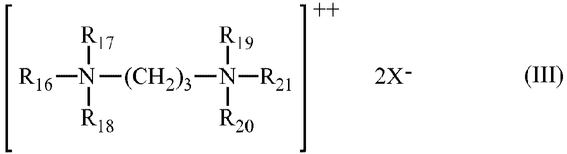 dans laquelle R₁₆ désigne un radical aliphatique comportant environ de 16 à 30 atomes de carbone, R₁₇, R₁₈, R₁₉, R₂₀ et R₂₁, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium ;
- les sels d'ammonium quaternaire contenant au moins une fonction ester, tels que ceux de formule (IV) suivante : 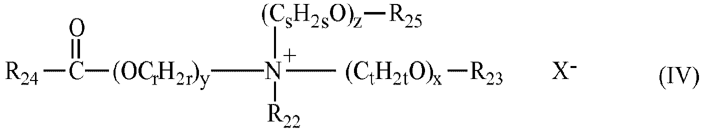 dans laquelle :
   R₂₂ est choisi parmi les radicaux alkyles en C₁-C₆ et les radicaux hydroxyalkyles ou dihydroxyalkyles en C₁-C₆ ;
   R₂₃ est choisi parmi :
      - le radical 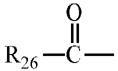
      - les radicaux R₂₇ hydrocarbonés en C₁-C₂₂, linéaires ou ramifiés, saturés ou insaturés,
      - l'atome d'hydrogène,
   R₂₅ est choisi parmi :
      - le radical 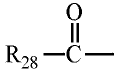
      - les radicaux R₂₉ hydrocarbonés en C₁-C₆, linéaires ou ramifiés, saturés ou insaturés,
      - l'atome d'hydrogène,
   R₂₄, R₂₆ et R₂₈, identiques ou différents, sont choisis parmi les radicaux hydrocarbonés en C₇-C₂₁, linéaires ou ramifiés, saturés ou insaturés ;
   r, s et t, identiques ou différents, sont des entiers valant de 2 à 6 ;
   y est un entier valant de 1 à 10 ;
   x et z, identiques ou différents, sont des entiers valant de 0 à 10 ;
   X⁻ est un anion simple ou complexe, organique ou inorganique ;
   sous réserve que la somme x + y + z vaut de 1 à 15, que lorsque x vaut 0 alors R₂₃ désigne R₂₇ et que lorsque z vaut 0 alors R₂₅ désigne R₂₉.

Les radicaux alkyles R₂₂ peuvent être linéaires ou ramifiés et plus particulièrement linéaires.

De préférence R₂₂ désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R₂₃ est un radical R₂₇ hydrocarboné, il peut être long et avoir de 12 à 22 atomes de carbone, ou court et avoir de 1 à 3 atomes de carbone.

Lorsque R₂₅ est un radical R₂₉ hydrocarboné, il a de préférence 1 à 3 atomes de carbone.

Avantageusement, R₂₄, R₂₆ et R₂₈, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.

Avantageusement, y est égal à 1.

De préférence, r, s et t, identiques ou différents, valent 2 ou 3, et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.
On utilise plus particulièrement dans la composition selon l'invention, les sels d'ammonium de formule (IV) dans laquelle :
- R₂₂ désigne un radical méthyle ou éthyle,
- x et y sont égaux à 1 ;
- z est égal à 0 ou 1 ;
- r, s et t sont égaux à 2 ;
- R₂₃ est choisi parmi :
   - le radical 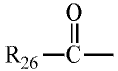
   - les radicaux méthyle, éthyle ou hydrocarbonés en C₁₄-C₂₂,
   - l'atome d'hydrogène ;
- R₂₅ est choisi parmi :
   - le radical 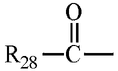
   - l'atome d'hydrogène ;
- R₂₄, R₂₆ et R₂₈, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.

Avantageusement, les radicaux hydrocarbonés sont linéaires.
On peut citer par exemple les composés de formule (IV) tels que les sels (chlorure ou méthylsulfate notamment) de diacyloxyéthyl-dimé-thylammonium, de diacyloxyéthyl-hydroxyéthyl-méthylammonium, de monoacyloxyéthyl-dihydroxyéthyl-méthylammonium, de triacyloxy-éthyl-méthylammonium, de monoacyloxyéthyl-hydroxyéthyl-diméthyl-ammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkyla-tion tel qu'un halogénure d'alkyle (méthyle ou éthyle de préférence), un sulfate de dialkyle (méthyle ou éthyle de préférence), le méthanesulfonate de méthyle, le para-toluènesulfonate de méthyle, la chlorhydrine du glycol ou du glycérol.

De tels composés sont par exemple commercialisés sous les dénominations DEHYQUART^{®} par la société HENKEL, STEPANQUAT^{®} par la société STEPAN, NOXAMIUM^{®} par la société CECA, REWOQUAT^{®} WE 18 par la société REWO-WITCO.

Les agents tensioactifs cationiques utilisés de manière particulièrement préférée dans l'invention sont choisis parmi les sels d'ammonium quaternaire comme le chlorure de béhényltriméthylammo-nium, les sels d'ammonium quaternaire contenant au moins une fonction ester comme le méthosulfate de distéaroyléthyl hydroxyéthyl méthylammonium, et leurs mélanges.

Lorsqu'au moins un desdits agents tensioactifs cationiques est utilisé, il(s) peut ou peuvent être utilisé(s) en une quantité allant de 0,1 à 10 % en poids, de préférence de 0,2 à 8% en poids, et plus particulièrement de 0,2 à 5 % en poids, par rapport au poids total de la composition.

Dans un mode de réalisation de l'invention, la composition peut comprendre en outre une ou plusieurs huiles végétales. A titre d'exemples d'huile végétale utilisable dans l'invention, on peut notamment citer l'huile de cameline, l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot et l'huile de calophyllum.

Lorsqu'au moins une desdites huiles végétales est présente, elle(s) peut ou peuvent être utilisée(s) en une quantité allant de 0,01 à 30% en poids, de préférence de 0,1 à 20% en poids, et plus particulièrement de 0,2 à 10% en poids, par rapport au poids total de la composition.

Selon un mode de l'invention, la composition peut comprendre en outre au moins une silicone.

Les silicones utilisables conformément à l'invention peuvent être solubles ou insolubles dans la composition. Elles peuvent être en particulier des polyorganosiloxanes insolubles dans la composition de l'invention et se présenter sous forme d'huiles, de cires, de résines ou de gommes.

Les silicones insolubles sont notamment dispersées dans les compositions sous forme de particules ayant généralement une taille moyenne en nombre comprise entre 2 nanomètres et 100 micromètres, de préférence entre 20 nanomètres et 20 micromètres (mesurée avec un granulomètre).

Les organopolysiloxanes sont définis plus en détail dans l'ouvrage de Walter NOLL "Chemistry and Technology of Silicones" (1968) Academic Press. Elles peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et, de préférence, 4 à 5. Il s'agit, par exemple, de l'octaméthylcyclotétra-siloxane commercialisé notamment sous le nom de "VOLATILE SILICONE 7207" par UNION CARBIDE ou "SILBIONE 70045 V 2" par RHODIA, le décaméthylcyclopentasiloxane commercialisé sous le nom de "VOLATILE SILICONE 7158" par UNION CARBIDE, "SILBIONE 70045 V 5" par RHODIA, ainsi que leurs mélanges.
   On peut également citer les cyclocopolymères du type diméthylsiloxane/méthylalkylsiloxane, tel que la "SILICONE VOLATILE FZ 3109" commercialisée par la société UNION CARBIDE, de structure chimique : 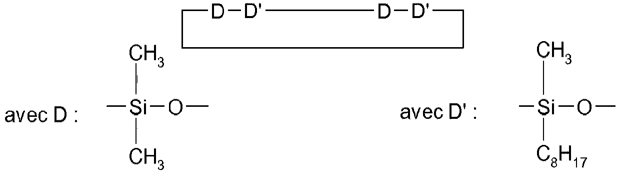
   On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

Parmi les silicones non volatiles, on peut notamment citer les polyalkylsiloxanes, les polyarylsiloxanes, les polyalkylarylsiloxanes, les gommes et les résines de silicones, les polyorganosiloxanes modifiés par des groupements organofonctionnels, les copolymères blocs linéaires polysiloxane(A)-polyoxyalkylène(B) de type (A-B)ₙ avec n >3 ; les polymères siliconés greffés, à squelette organique non siliconé, constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromonomère polysiloxane ; les polymères siliconés greffés, à squelette polysiloxanique greffé par des monomères organiques non siliconés, comprenant une chaîne principale de polysiloxane sur laquelle se trouve greffé, à l'intérieur de ladite chaîne ainsi qu'éventuellement à l'une au moins de ses extrémités, au moins un macromonomère organique ne comportant pas de silicone ; ainsi que leurs mélanges.

A titre d'exemples de polyalkylsiloxanes, on peut notamment citer les polydiméthylsiloxanes à groupements terminaux triméthylsilyle ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHONE POULENC telles que par exemple l'huile 70 047 V 500 000 ;
- les huiles de la série MIRASIL commercialisées par la société RHONE POULENC ;
- les huiles de la série 200 de la société DOW CORNING telles que plus particulièrement la DC200 de viscosité 60 000 cSt ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHONE POULENC.

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les polyalkylarylsiloxanes peuvent être notamment choisis parmi les polydiméthyl-méthylphénylsiloxanes, les polydiméthyl-diphényl-siloxanes linéaires et/ou ramifiés de viscosité de 1.10⁻⁵ à 5.10² m²/s à 25°C.

Parmi ces polyalkylarylsiloxanes, on peut citer à titre d'exemple les produits commercialisés sous les dénominations suivantes :
- les huiles SILBIONE de la série 70 641 de RHONE POULENC ;
- les huiles des séries RHODORSIL 70 633 et 763 de RHONE POULENC ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000, utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- les gommes polydiméthylsiloxane,
- les gommes polydiméthylsiloxanes/méthylvinylsiloxane,
- les gommespolydiméthylsiloxane/diphénylsiloxane,
- les gommes polydiméthylsiloxane/phénylméthylsiloxane,
- les gommes polydiméthylsiloxane / diphénylsiloxane / méthylvinylsiloxane.

Des silicones que l'on peut utiliser dans la composition selon l'invention sont des mélanges tels que :
- les mélanges formés à partir d'un polydiméthylsiloxane hydroxylé en bout de chaîne (dénommé diméthiconol selon la nomenclature du dictionnaire CTFA) et d'un polydiméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 20 et 85 % d'une huile SF 96.

Les résines d'organopolysiloxanes utilisables conformément à l'invention sont des systèmes siloxaniques réticulés renfermant les unités : R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle.

Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone-copolyol commercialisé par la société DOW CORNING sous la dénomination DC 1248 ou les huiles SILWET^{®} L 722, L 7500, L 77, L 711 de la société UNION CARBIDE et l'alkyl(C₁₂)-méthicone-copolyol commercialisée par la société DOW CORNING sous la dénomination Q2 5200 ;
- des groupements aminés substitués ou non, comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements ammonium quaternaires comme les produits commercialisés sous les dénominations ABILQUAT 3272 et ABILQUAT 3474 par la société GOLDSCHMIDT ;
- des groupements thiols, comme les produits commercialisés sous les dénominations "GP 72 A" et "GP 71" de GENESEE ;
- des groupements alcoxylés, comme le produit commercialisé sous la dénomination "SILICONE COPOLYMER F-755" par SWS SILICONES et ABIL WAX^{®} 2428, 2434 et 2440 par la société GOLDSCHMIDT ;
- des groupements hydroxylés, comme les polyorganosiloxanes à fonction hydroxyalkyle décrits dans la demande de brevet français FR-A-85 16334 ;
- des groupements acyloxyalkyle tels que, par exemple, les polyorganosiloxanes décrits dans le brevet US-A-4957732 ;
- des groupements anioniques du type acide carboxylique comme, par exemple, dans les produits décrits dans le brevet EP 186 507 de la société CHISSO CORPORATION, ou du type alkyl-carboxylique comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABlL^{®} S201" et "ABIL^{®} S255".
- des groupements hydroxyacylamino, comme les polyorganosiloxanes décrits dans la demande EP 342 834. On peut citer, par exemple, le produit Q2-8413 de la société DOW CORNING.

Les silicones particulièrement préférées dans l'invention sont les polydiméthylsiloxanes non substitués et les silicones aminées.

Lorsqu'au moins une desdites silicones est présente, elle(s) est (ou sont) contenue(s) de préférence en une quantité allant de 0,05 à 20 % en poids, plus particulièrement de 0,1 à 10 % en poids, et mieux encore de 0,2 à 5 % en poids par rapport au poids total de la composition.

La composition utilisée dans l'invention peut comprendre en outre un ou plusieurs composés naturels ou d'origine naturelle, tels que des actifs et extraits naturels comme des jus de fruits, des huiles essentielles et des extraits de plantes.

Par « composé naturel », on entend un produit que l'on obtient de la terre ou du sol, ou à partir de végétaux ou d'animaux, via éventuellement un ou des processus physiques comme, par exemple, un broyage, un raffinage, une distillation, une purification ou une filtration.

Par « composé d'origine naturelle », on entend un composé naturel ayant subi un ou plusieurs traitements chimiques n'affectant pas les qualités essentielles de ce composé et/ou un composé comprenant majoritairement des composés naturels.

La composition utilisée dans l'invention peut également comprendre en outre un ou plusieurs additifs classiques bien connus dans la technique, tels que des tensio-actifs anioniques, non ioniques ou amphotères, des polymères anioniques, cationiques, non-ioniques, ou amphotères, des polyols, des protéines, des vitamines, des agents réducteurs, des plastifiants, des adoucissants, des agents anti-mousse, des agents hydratants, des pigments, des argiles, des charges minérales, des filtres UV, des colloïdes minéraux, des peptisants, des solubilisants, des parfums, des conservateurs, des agents nacrants, des propulseurs, et des épaississants minéraux, ou leurs mélanges.

L'homme de métier veillera à choisir les éventuels additifs et leur quantité de manière à ce qu'ils ne nuisent pas aux propriétés des compositions utilisées dans la présente invention.

Ces additifs sont généralement présents dans la composition selon l'invention en une quantité allant de 0 à 20 % en poids par rapport au poids total de la composition.

Les compositions utilisées dans l'invention peuvent se présenter sous la forme d'une lotion plus ou moins épaissie, d'une crème, d'un gel ou d'une émulsion.

L'invention est également relative à un procédé de traitement cosmétique des matières kératiniques, de préférence des fibres kératiniques comme les cheveux, qui consiste à appliquer une quantité efficace d'une composition cosmétique telle que décrite ci-dessus, sur lesdites matières, à éventuellement rincer après un éventuel temps de pause.

Lorsque l'on applique la composition selon l'invention sous forme d'une lotion ou d'une crème, on la laisse éventuellement pauser sur les cheveux pendant environ 1/2 min. à 5 minutes, puis on rince éventuellement à l'eau.

Les exemples suivants sont donnés à titre illustratif de la présente invention.

Dans les exemples suivants, toutes les quantités sont indiquées en pour cent en poids de matière active par rapport au poids total de la composition, sauf indication contraire.

### EXEMPLES

### Exemple 1

On a préparé les compositions A et B suivantes à partir des ingrédients indiqués dans le tableau ci-dessous.

| Compositions | A (comparative) | B (invention) |
|---|---|---|
| Alcool cétéarylique⁽¹⁾ | 3 | 3 |
| Esters cétyliques (Mélange myristate/palmitate/ stéarate de myristyle/cétyle/stéaryle)⁽²⁾ | 0,5 | 0,5 |
| Méthosulfate de distéaroyléthyl hydroxyéthyl-méthylammonium/ Alcool cétéarylique (75/25 en poids)⁽³⁾ | 3 | 3 |
| Huile de noyau d'abricot | 1 | 1 |
| Amidon de maïs (zea mays starch)⁽⁴⁾ | 0,5 | 0,5 |
| Mélange majoritairement constitué de n-undécane et de n-tridécane⁽⁵⁾ | - | 3 |
| Conservateurs | 0,35 | 0,35 |
| Parfum | 0,6 | 0,6 |
| Eau qsp | 100 | 100 |
| ⁽¹⁾ vendu sous la dénomination commerciale Lanette O OR par la société Cognis | | |
| ⁽²⁾ vendu sous la dénomination commerciale Miraceti par la société Laserson | | |
| ⁽³⁾ vendu sous la dénomination commerciale Dehyquart F 75 par la société Cognis | | |
| ⁽⁴⁾ vendu sous la dénomination commerciale C*Gel 04201 par la société CARGILL | | |
| ⁽⁵⁾ selon l'exemple 2 de WO 2008/155059 | | |

On a appliqué les compositions A et B sur les cheveux, et comparé les propriétés obtenues. On a observé un lissage nettement meilleur au rinçage et sur cheveux humides avec la composition B, par rapport à ceux observés pour la composition comparative A.

En outre, les cheveux sont plus toniques et légers au rinçage et sur cheveux humides avec la composition B selon l'invention.

### Exemple 2

On a préparé les compositions C et D suivantes à partir des ingrédients indiqués dans le tableau ci-dessous.

| Compositions | c | D |
|---|---|---|
| Alcool cétéarylique⁽¹⁾ | 3 | 3 |
| Méthosulfate de distéaroyléthyl hydroxyéthyl-diméthylammonium/ Alcool cétéarylique (75/25 en poids) (2) | 4 | - |
| Chlorure de béhényltriméthylammium à 80% de matière active | - | 2 |
| Amodimethicone en émulsion aqueuse cationique à 60 %⁽³⁾ | - | 1,5 |
| Hydroxyéthylcellulose⁽⁴⁾ | 0,2 | - |
| Hydroxypropylguar⁽⁵⁾ | - | 0,3 |
| Mélange majoritairement constitué de n-undécane et de n-tridécane⁽⁶⁾ | - | 2 |
| Mélange de n-dodécane et de n-tétradécane⁽⁷⁾ | 6 | - |
| Conservateurs | 0,35 | 0,35 |
| Parfum | 0,6 | 0,6 |
| Eau qsp | 100 | 100 |
| ⁽¹⁾ vendu sous la dénomination commerciale Lanette O OR par la société Cognis | | |
| ⁽²⁾ vendu sous la dénomination commerciale Dehyquart F 75 par la société Cognis | | |
| ⁽³⁾ vendu sous la dénomination commerciale Dow Corning 2-8299 Cationic emulsion par la société Dow Corning | | |
| ⁽⁴⁾ vendu sous la dénomination commerciale NATROSOL 250 HHR par la société AQUALON | | |
| ⁽⁵⁾ vendu sous la dénomination commerciale JAGUAR HP 105 par la société RHODIA | | |
| ⁽⁶⁾ selon l'exemple 2 de WO 2008/155059 | | |
| ⁽⁷⁾ vendu sous la dénomination commerciale Vegelight 1214 par la société Biosynthis | | |

On a appliqué les compositions C et D sur les cheveux. On a observé un excellent lissage au rinçage et sur cheveux humides. En outre, les cheveux présentaient d'excellentes tonicité et légèreté au rinçage et sur cheveux humides.

## Revendications

1. Utilisation pour le traitement cosmétique des fibres kératiniques comme les cheveux, d'une composition cosmétique comprenant, dans un milieu cosmétiquement acceptable :
- de 1,5 à 10% en poids par rapport au poids total de la composition, d'au moins deux alcanes linéaires volatils distincts différant entre eux d'un nombre de carbones n d'au moins 1,
- un ou plusieurs alcools gras solides et
- un ou plusieurs agents épaississants polymères à motifs sucres.

2. Utilisation selon la revendication 1, **caractérisée en ce que** les alcanes linéaires volatils sont des alcanes linéaires comportant de 7 à 15 atomes de carbone.

3. Utilisation selon la revendication 2, **caractérisée en ce que** les alcanes linéaires volatils sont choisis parmi le n-nonane, le n-undécane, le n-dodécane, le n-tridécane, le n-tétradécane, et leurs mélanges.

4. Utilisation selon l'une quelconque des revendications précédentes, **caractérisée en ce que** les alcanes linéaires volatils sont présents en une quantité allant de 2 à 10 % en poids par rapport au poids total de la composition.

5. Utilisation selon l'une quelconque des revendications précédentes, **caractérisée en ce que** l'alcool gras solide est un alcool gras linéaire en C₁₂₋₃₀.

6. Utilisation selon l'une quelconque des revendications précédentes, **caractérisée en ce que** le ou les alcools gras solides sont utilisés en une teneur allant de 0,5 % à 20 % en poids de préférence de 1 à 15% en poids, et plus particulièrement de 2 à 10% en poids par rapport au poids total de la composition.

7. Utilisation selon l'une quelconque des revendications précédentes, **caractérisée en ce que** l'agent épaississant polymère à motifs sucres est choisi parmi les gommes natives telles que :
a) les exsudats d'arbres ou d'arbustes comme la gomme arabique, la gomme ghatti, la gomme karaya, la gomme tragacanthe (ou adragante),
b) les gommes issues d'algues comme l'agar, les alginates, les carraghénanes et les furcelleranes,
c) les gommes issus de semences ou tubercules comme la gomme de guar, la gomme de caroube, la gomme de fenugrec, la gomme de tamarin, la gomme de konjac, l'amidon (polymère du glucose),
d) les gommes microbiennes comme la gomme de xanthane, la gomme de gellane, la gomme de scléroglucane,
e) les extraits de plantes comme la cellulose (polymère du glucose, motif de répétition cellobiose) et l'amidon (polymère du glucose, motifs de répétition amylose et amylopectine),
non modifiés ou modifiées par voie physique ou chimique.

8. Utilisation selon l'une quelconque des revendications précédentes, **caractérisée en ce que** l'agent épaississant polymère à motifs sucres est non ionique, cationique, anionique ou amphotère et de préférence non ionique ou cationique.

9. Utilisation selon l'une quelconque des revendications précédentes, **caractérisée en ce que** l'agent épaississant polymère à motifs sucres est choisi parmi les gommes de guar non-ioniques modifiées par des groupements hydroxylakyle en C₁-C₆, les gommes de guar modifiées par des groupements cationiques, la gomme de caroube modifiée par des groupements hydroxypropyltrimmonium, les amidons prégélatinisés et/ou oxydés et/ou réticulés et/ou estérifiés, les amidons amphotères, les éthers de celluloses, les esters de celluloses et les esters éthers de celluloses, et leurs mélanges.

10. Utilisation selon l'une quelconque des revendications précédentes, **caractérisée en ce que** le ou les agent(s) épaississant(s) polymère(s) à motifs sucres sont utilisés en une teneur allant de 0,01 à 10 % en poids, plus préférentiellement de 0,1 à 5 % en poids et mieux encore de 0,2 à 3 % en poids par rapport au poids total de la composition.

11. Utilisation selon l'une quelconque des revendications précédentes, **caractérisée en ce que** la composition cosmétique comprend au moins un agent tensioactif cationique.

12. Utilisation selon la revendication 11, **caractérisée en ce que** le ou les agent(s) tensioactif(s) cationique(s) sont utilisés en une teneur allant de 0,1 à 10% en poids, et plus particulièrement de 0,2 à 8% en poids par rapport au poids total de la composition.

13. Utilisation selon l'une quelconque des revendications précédentes, **caractérisée en ce que** la composition comprend au moins une huile végétale, de préférence choisie parmi l'huile de cameline, l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot, l'huile de calophyllum et leurs mélanges.

14. Utilisation selon l'une quelconque des revendications précédentes, **caractérisée en ce que** la composition comprend au moins une silicone.

15. Procédé de traitement cosmétique des matières kératiniques, de préférence des fibres kératiniques comme les cheveux, mettant en œuvre une composition telle que définie dans l'une quelconque des revendications 1 à 14.

## Patentansprüche

1. Verwendung einer Kosmetikzusammensetzung, die in einem kosmetisch unbedenklichen Medium Folgendes umfasst, für die kosmetische Behandlung von Keratinfasern wie dem Haar:
- 1,5 Gew.-% bis 10 Gew.-% in Bezug auf das Gesamtgewicht der Zusammensetzung an mindestens zwei unterschiedlichen flüchtigen geradkettigen Alkanen, die sich voneinander in der Anzahl der Kohlenstoffe n um mindestens 1 unterscheiden,
- einen oder mehrere feste Fettalkohole und
- ein oder mehrere polymere Verdickungsmittel mit Zuckereinheiten.

2. Verwendung nach Anspruch 1, **dadurch gekennzeichnet, dass** es sich bei den flüchtigen geradkettigen Alkanen um geradkettige Alkane mit 7 bis 15 Kohlenstoffatomen handelt.

3. Verwendung nach Anspruch 2, **dadurch gekennzeichnet, dass** die flüchtigen geradkettigen Alkane aus der Reihe n-Nonan, n-Undecan, n-Dodecan, n-Tridecan, n-Tetradecan und ihren Mischungen ausgewählt sind.

4. Verwendung nach einem der vorhergehenden Ansprüche, **dadurch gekennzeichnet, dass** die flüchtigen geradkettigen Alkane in einer Menge von 2 bis 10 Gew.-%, in Bezug auf das Gesamtgewicht der Zusammensetzung vorliegen.

5. Verwendung nach einem der vorhergehenden Ansprüche, **dadurch gekennzeichnet, dass** es sich bei dem festen Fettalkohol um einen geradkettigen C₁₂-C₃₀-Fettalkohol handelt.

6. Verwendung nach einem der vorhergehenden Ansprüche, **dadurch gekennzeichnet, dass** der feste Fettalkohol oder die festen Fettalkohole in einem Gehalt von 0,5 Gew.-% bis 20 Gew.-%, vorzugsweise 1 bis 15 Gew.-%, insbesondere 2 bis 10 Gew.-%, in Bezug auf das Gesamtgewicht der Zusammensetzung eingesetzt werden.

7. Verwendung nach einem der vorhergehenden Ansprüche, **dadurch gekennzeichnet, dass** das polymere Verdickungsmittel mit Zuckereinheiten aus der Reihe der nativen Gummen wie den folgenden ausgewählt ist:
a) Baum- oder Strauchabsonderungen wie Gummi arabicum, Ghatti-Gummi, Karaya-Gummi, Traganth-Gummi (auch "Adragant"-Gummi genannt),
b) Gummen, die aus Algen stammen, wie Agar, Alginaten, Carrageenan-Arten und Furcelleran-Arten,
c) Gummen, die aus Samen oder Knöllchen stammen, wie Guar-Gummi, Karoben-Gummi, Boxhornkleegummi, Tamarindengummi, Konjac-Gummi, Stärke (Glukosepolymer),
d) mikrobiellen Gummen wie Xanthangummi, Gellangummi, Skleroglucangummi,
e) Pflanzenextrakten wie Cellulose (Glukosepolymer, Wiederholungseinheit Cellobiose) und Stärke (Glukosepolymer, Wiederholungseinheiten Amylose und Amylopectin),
die unmodifiziert oder auf physikalischem oder chemischem Weg modifiziert sind.

8. Verwendung nach einem der vorhergehenden Ansprüche, **dadurch gekennzeichnet, dass** das polymere Verdickungsmittel mit Zuckereinheiten nichtionisch, kationisch, anionisch oder amphoter und vorzugsweise nichtionisch oder kationisch ist.

9. Verwendung nach einem der vorhergehenden Ansprüche, **dadurch gekennzeichnet, dass** das polymere Verdickungsmittel mit Zuckereinheiten aus der Reihe der nichtionischen mit C₁-C₆-Hydroxyalkylgruppen modifizierten Guar-Gummen, der mit kationischen Gruppierungen modifizierten Guar-Gummen, mit Hydroxypropyltrimonium modifiziertem Karobengummi, der vorverkleisterten und/oder oxidierten und/oder vernetzten und/oder veresterten Stärken, der amphoteren Stärken, der Celluloseether, der Celluloseester und der Celluloseetherester und ihren Mischungen ausgewählt ist.

10. Verwendung nach einem der vorhergehenden Ansprüche, **dadurch gekennzeichnet, dass** das bzw. die polymere(n) Verdickungsmittel mit Zuckereinheiten in einem Gehalt von 0,01 bis 10 Gew.-%, stärker bevorzugt 0,1 bis 5 Gew.-%, noch besser 0,2 bis 3 Gew.-%, in Bezug auf das Gesamtgewicht der Zusammensetzung eingesetzt wird bzw. werden.

11. Verwendung nach einem der vorhergehenden Ansprüche, **dadurch gekennzeichnet, dass** die Kosmetikzusammensetzung mindestens ein kationisches Tensid umfasst.

12. Verwendung nach Anspruch 11, **dadurch gekennzeichnet, dass** das bzw. die kationische(n) Tensid(e) in einem Gehalt von 0,1 bis 10 Gew.-%, insbesondere 0,2 bis 8 Gew.-%, in Bezug auf das Gesamtgewicht der Zusammensetzung eingesetzt werden.

13. Verwendung nach einem der vorhergehenden Ansprüche, **dadurch gekennzeichnet, dass** die Zusammensetzung mindestens ein Pflanzenöl, vorzugsweise ausgewählt aus Leindotteröl, Süßmandelöl, Avocadoöl, Rizinusöl, Olivenöl, Jojobaöl, Sonnenblumenöl, Weizenkeimöl, Sesamöl, Erdnussöl, Traubenkernöl, Sojaöl, Rapsöl, Distelöl, Kopraöl, Maisöl, Haselnussöl, Karite-Butter, Palmöl, Aprikosenkernöl, Calophyllum-Öl und ihren Mischungen umfasst.

14. Verwendung nach einem der vorhergehenden Ansprüche, **dadurch gekennzeichnet, dass** die Zusammensetzung mindestens ein Silikon umfasst.

15. Verfahren zur kosmetischen Behandlung von Keratinsubstanzen, vorzugsweise Keratinfasern wie dem Haar, wobei eine Zusammensetzung wie in einem der Ansprüche 1 bis 14 definiert eingesetzt wird.

## Claims

1. Use for the cosmetic treatment of keratin fibres, such as the hair, of a cosmetic composition comprising, in a cosmetically acceptable medium:
- from 1.5% to 10% by weight, relative to the total weight of the composition, of at least two different volatile linear alkanes differing from each other by a carbon number n of at least **1,**
- one or more solid fatty alcohols and
- one or more polymeric thickeners containing sugar units.

2. Use according to Claim 1, **characterized in that** the volatile linear alkanes are linear alkanes comprising from 7 to 15 carbon atoms.

3. Use according to Claim 2, **characterized in that** the volatile linear alkanes are chosen from n-nonane, n-undecane, n-dodecane, n-tridecane and n-tetradecane, and mixtures thereof.

4. Use according to any one of the preceding claims, **characterized in that** the volatile linear alkanes are present in an amount ranging from 2% to 10% by weight relative to the total weight of the composition.

5. Use according to any one of the preceding claims, **characterized in that** the solid fatty alcohol is a linear C₁₂₋₃₀ fatty alcohol.

6. Use according to any one of the preceding claims, **characterized in that** the solid fatty alcohol(s) are used in a content ranging from 0.5% to 20% by weight, preferably from 1% to 15% by weight and more particularly from 2% to 10% by weight relative to the total weight of the composition.

7. Use according to any one of the preceding claims, **characterized in that** the polymeric thickener containing sugar units is chosen from native gums such as:
a) tree or shrub exudates, such as gum arabic, ghatti gum, karaya gum or gum tragacanth (or tragacanth),
b) gums derived from algae, such as agar, alginates, carrageenans and furcellerans,
c) gums derived from seeds or tubers, such as guar gum, locust bean gum, fenugreek gum, tamarind gum, konjac gum and starch (glucose polymer),
d) microbial gums, such as xanthan gum, gellan gum or scleroglucan gum,
e) plant extracts, such as cellulose (glucose polymer, cellobiose repeating unit) and starch (glucose polymer, amylose and amylopectin repeating units),
which are unmodified or physically or chemically modified.

8. Use according to any one of the preceding claims, **characterized in that** the polymeric thickener containing sugar units is nonionic, cationic, anionic or amphoteric and preferably nonionic or cationic.

9. Use according to any one of the preceding claims, **characterized in that** the polymeric thickener containing sugar units is chosen from nonionic guar gums modified with C₁-C₆ hydroxyalkyl groups, guar gums modified with cationic groups, locust bean gum modified with hydroxypropyltrimonium groups, pregelatinized and/or oxidized and/or crosslinked and/or esterified starches, amphoteric starches, cellulose ethers, cellulose esters and cellulose ester ethers, and mixtures thereof.

10. Use according to any one of the preceding claims, **characterized in that** the polymeric thickener(s) containing sugar units are used in a content ranging from 0.01% to 10% by weight, more preferentially from 0.1% to 5% by weight and better still from 0.2% to 3% by weight relative to the total weight of the composition.

11. Use according to any one of the preceding claims, **characterized in that** the cosmetic composition comprises at least one cationic surfactant.

12. Use according to Claim 11, **characterized in that** the cationic surfactant(s) are used in a content ranging from 0.1% to 10% by weight and more particularly from 0.2% to 8% by weight relative to the total weight of the composition.

13. Use according to any one of the preceding claims, **characterized in that** the composition comprises at least one plant oil, preferably chosen from camelina oil, sweet almond oil, avocado oil, castor oil, olive oil, jojoba oil, sunflower oil, wheatgerm oil, sesame oil, groundnut oil, grapeseed oil, soybean oil, rapeseed oil, safflower oil, coconut oil, corn oil, hazelnut oil, shea butter, palm oil, apricot kernel oil and beauty-leaf oil, and mixtures thereof.

14. Use according to any one of the preceding claims, **characterized in that** the composition comprises at least one silicone.

15. Cosmetic process for treating keratin materials, preferably keratin fibres such as the hair, using a composition as defined in any one of Claims 1 to 14.
